# EUROPÄISCHE PATENTSCHRIFT

(11) **EP 2 433 665 B1**
(45) Veröffentlichungstag und Bekanntmachung des Hinweises auf die Patenterteilung: **07.08.2019**
(21) Anmeldenummer: 11194615.8
(22) Anmeldetag: 11.03.2009
(51) Int. Cl.: A61M 15/00

(54) **Inhalator und Sieb für einen Inhalator**
Inhaler and sieve for an inhaler
Inhalateur et filtre pour un inhalateur

(30) Priorität: 13.03.2008 DE 102008014025
(43) Veröffentlichungstag der Anmeldung: 28.03.2012
(62) Teilanmeldung aus: 09719496.3
(73) Patentinhaber: Boehringer Ingelheim Pharma GmbH & Co. KG, 55216 Ingelheim am Rhein (DE)
(72) Erfinder: Haerder, Lukas, 55216 INGELHEIM AM RHEIN (DE); Breuer, Claus, 55216 INGELHEIM AM RHEIN (DE)
(74) Vertreter: Simon, Elke Anna Maria

(56) Entgegenhaltungen:
- EP-A1- 0 268 752
- WO-A-2004/047796
- WO-A1-2007/118801
- US-A- 2 569 720
- US-A- 3 653 380
- US-A- 5 797 391

## Beschreibung

Die Erfindung betrifft zunächst einen Inhalator fürpulverförmige, insbesondere medizinische Substanzen, mit einem zu einem Mundstück führenden Saugluftkanal, weiter einem in einer Aufnahmekammer bevorzugt beweglich vorgesehenen Substanz-Vorratsbehältnis und einem in dem Saugluftkanal zwischen der Aufnahmekammer und dem Mundstück angeordneten Siebteil, das einen Einspannrand aufweist und einem in einem Querschnitt innerhalb des Einspannrandes verlaufenden Siebbereich, wobei der Siebbereich einen zu einer Seite hinausladend geformten Vorstandsbereich aufweist.

Derartige Inhalatoren sind bekannt. Es wird bspw. auf die WO 2004/062716 A1 und WO 2007/118801 A1 verwiesen. Das Siebteil begrenzt einseitig, Richtung Mundstück, die Aufnahmekammer. Hiermit ist es aber auch im Hinblick auf die gewünschten sehr genauen Abmessungen der Aufnahmekammer, was wiederum mit der gewünschten Beweglichkeit des Substanz-Vorratsbehältnisses zusammenhängt, Anforderungen an eine hochpräzise Maßhaltigkeit unterworfen. Bei einem dem internen Stand der Technik der Anmelderin entsprechenden Siebteil ist eine kuppelförmige Auswölbung als Vorstandsbereich vorgesehen. Die Auswölbung ist zu dem Substanz-Vorratsbehältnis hin ausgebildet. Es hat sichjedoch herausgestellt, dass fertigungstechnisch die Einleitung der erforderten Maßtoleranzen sehr schwierig ist.

Ältere Beispiele für Inhalatoren, bei denen sich ein flaches Gitter, Sieb oder Schirm zwischen Pulverkapsel und Einatemöffnung bzw. Mundstück befinden, sind in US 5797391, US 2569720 und WO 2004/047796 A2 offenbart.

Ausgehend von dem dargestellten Stand der Technik beschäftigt sich die Erfindung mit der Aufgabe, einen Inhalator anzugeben, dessen Siebteil vorteilhaft gestaltet ist.

Eine mögliche Lösung der Aufgabe ist nach einem ersten Erfindungsgedanken durch den Gegenstand des Anspruches 1 gegeben.
Ein weiterer Erfindungsaspekt ist darauf abgestellt, dass der Vorstandsbereich eine Abflachung aufweist. Überraschend hat sich herausgestellt, dass im Sinne einer erhöhten Maßhaltigkeit zwar weiter eine Ausbildung eines Vorstandsbereiches im Siebbereich des Siebteils möglich ist; dass die Maßhaltigkeit aber schon dadurch entscheidend verbessert werden kann, dass dieser Vorstandsbereich eine Abflachung aufweist. Der Vorstandsbereich ist also nicht kontinuierlich kuppelförmig gestaltet. Der Vorstandsbereich wächst vielmehr zunächst aus dem umgebenden Siebbereich heraus, knickt dann aber gleichsam, in einer Querschnittsdarstellung, im Sinne einer Abflachung ab.

Weitere Merkmale der Erfindung sind nachstehend, auch in der Figurenbeschreibung, oftmals in ihrer bevorzugten Zuordnung zu dem vorstehend behandelten Anspruchskonzept erläutert. Sie können aber auch in einer Zuordnung zu nur einer oder mehreren einzelnen Merkmalen dieses Anspruches oder unabhängig bzw. in einem anderen Gesamtkonzept von Bedeutung sein.

In der Querschnittsbetrachtung ragt der Vorstands bereich quer zu einer durch den Einspannrand verlaufenden Ebene über den Einspannrand hinaus.

Der Einspannrand als solcher weist eine Abwinklung auf. Die Abwinklung kann in Richtung des Vorstandsbereiches vorgesehen sein, aber auch entgegengesetzt hierzu. Die Abwinklung ist durch einen äußersten Randabschnitt des Siebteils gebildet. Das Siebteil kann insgesamt aus einem Drahtgewebe- Flachstück durch Umbiegen bzw. Tiefziehen, unter zumindest teilweise plastischer Verformung, hergestellt sein.

Weiter ist bevorzugt, dass die Abflachung in der Querschnittsbetrachtung mittig des Siebbereiches ist. Diese mittige Anordnung bezieht sich insbesondere auf ein Siebteil, das insgesamt einen kreisförmigen Grundriss aufweist. Es kann aber auch bei einem eckigen Grundriss umgebend zu dem hierbei gegebenen Mittelpunkt vorgesehen sein.

Die Abflachung als Solche hat eine Erstreckung bezogen auf eine Querschnittsdarstellung, die einem Teil des gesamten freien Abstandes zwischen gegenüberliegenden Bereichen des Einspannrandes entspricht. Bei einem kreisförmigen Durchmesser also einem Teil eines insoweit gegebenen Durchmessermaßes, Dieser Teilbereich entspricht bevorzugt 5% oder mehr des freigespannten Siebes innerhalb des Einspannrandes. Hierbei im Einzelnen bezogen auf eine Projektion einer Linie, die die gegenüberliegenden Bereiche des Einspannrandes unmittelbar miteinander verbindet. Im Falle eines rechteckigen Grundrisses ist diese Maßangabe zunächst auf die kleinste Abmessung zwischen gegenüberliegenden Bereichen des Einspannrandes bezogen. Weiter bevorzugt beträgt das Maß weniger als 15% der Gesamtabmessung der genannten Querschnittslinie, Hierbei sind, soweit eine Einschränkung auf den durch die obere und untere Schranke nunmehr genannten Bereich erfolgt, bezüglich dieses Bereiches auch alle Zwischenwerte in die Offenbarung eingeschlossen, und zwar insbesondere in 1/10%-Schritten. Die genannten Abmessungen beziehen sich auf eine Gesamtabmessung des Siebbereiches in dem angesprochenen freigespannten Bereich zwischen 5 und 15 mm. Auch diesbezüglich sind alle Zwischenwerte, insbesondere in 1/10 mm-Schritten, in die Offenbarung eingeschlossen.

Das Sieb selbst besteht bevorzugt aus metallischen Drähten. Als Werkstoff kommt insbesondere ein Edelstahlwerkstoff in Betracht, vorzugsweise mit Legierungsbestandteilen Chrom und/oder Nickel, wobei weiter bevorzugt der Chromanteil doppelt so hoch oder höher als der Nickelanteil ist.

Das Sieb besteht im Einzelnen geeigneterweise aus einem Gewebe aus den genannten Drähten. Es kann eine Maschenweite von 0,4 oder mehr Millimetern aufweisen. Weiter bevorzugt eine Maschenweite von 1,5 mm oder weniger.

Darüber hinaus bevorzugt eine Maschenweite im Bereich von 0,9-1 mm. In dem genannten Bereich von 0,m4-1,5 mm sind hiermit auch sämtliche Zwischenwerte, und zwar insbesondere in 1/10mm-Schritten von der unteren und/oder oberen Grenze auf die jeweils andere Grenze hin einbezogen. "Und" steht hierbei dafür, dass beide Grenzen um jeweils ein bzw, mehrere Zehntel auf die jeweils andere Grenze hin verschoben, d.h. eingegrenzt werden.

Der Draht selbst kann bevorzugt einen Durchmesser zwischen 0,1 und 0,5 mm aufweisen, wobei auch hier jegliche Zwischenwerte, insbesondere in 1/10mm-Schritten, in die Offenbarung mit einbezogen sind.

Im Weiteren betrifft die Erfindung ein Siebteil für einen Inhalator, insbesondere einen Inhalator in einer der Ausgestaltungen, wie er vorstehend beschrieben worden ist, wobei das Siebteil einen Einspannrand und einen in einem Querschnitt innerhalb des Einspannrandes verlaufenden Siebbereich aufweist, wobei weiter der Siebbereich einen zu einer Seite hin ausladend geformten Vorstandsbereich aufweist.

Bezüglich des Siebteils stellt sich die Aufgabe, dieses im Hinblick auf einen Einsatz in einem Inhalator, insbesondere einem Pulverinhalator, günstig zu gestalten.

Eine mögliche Lösung dieser Aufgabe ist beim Gegenstand des Anspruches 11 gegeben, wobei in diesem Fall darauf abgestellt ist, dass der Vorstandsbereich eine Abflachung aufweist. Zu den hiermit insbesondere im Zusammenhang mit einem Pulverinhalator erzielbaren Vorteilen wird auch auf die Ausführungen eingangs bezüglich des Inhalators insgesamt verwiesen. Desgleichen auch bezüglich der üblichen Ausgestaltungen des Siebteils.

Nachstehend ist die Erfindung des Weiteren anhand der beigefügten Zeichnung, die jedoch lediglich ein Ausführungsbeispiel darstellt, erläutert. Hierbei zeigt:
- Fig. 1: einen Inhalator mit im Saugluftkanal angeordnetem Sieb;
- Fig. 2: eine Herausvergrößerung des Bereichs II in Fig. 1;
- Fig. 3: eine Darstellung gemäß Fig. 2, jedoch mit abweichend gestaltetem Einspannrand des Siebteils;
- Fig. 4: einen Querschnitt durch das Siebteil alleine;
- Fig. 5: eine Draufsicht auf das Siebteil gemäß Fig. 4 in der Ansicht von oben;
- Fig. 6: eine Darstellung gemäß Fig. 4, jedoch mit abweichend gestaltetem Einspannrand;
- Fig. 7: eine Draufsicht auf den Gegenstand gemäß Fig, 6, gesehen von oben.

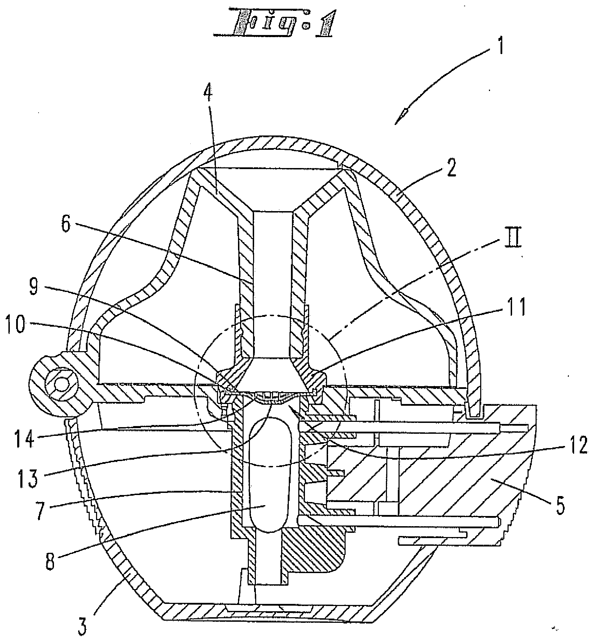

Mit Bezug zu Fig. 1 ist ein Pulverinhalator im Querschnitt dargestellt, wie grundsätzlich etwa aus der bereits eingangs genannten WO 2004/062716 A1 bekannt ist.

Der Inhalator 1 weist ein Abdeckteil 2 auf, eine Aufnahmegehäuse 3, ein Mundstück 4 und eine Betätigungstaste 5.

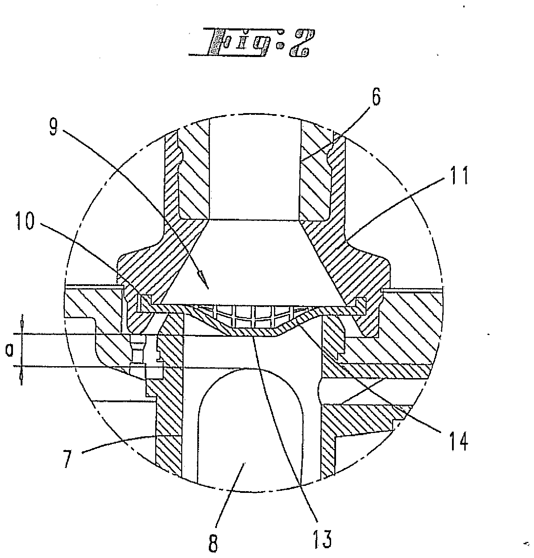

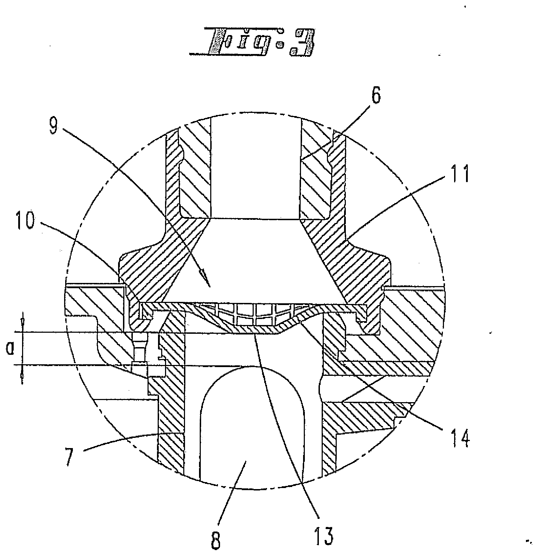

An das Mundstück 4 schließt sich innenseitig ein Saugluftkanal 6 an, der in eine Aufnahmekammer 7 übergeht, in welchem sich ein Substanzvorratsbehältnis 8 befindet. Zwischen der Aufnahmekammer 7 und dem Saugluftkanal 6 ist ein Sieb teil 9 angeordnet, das vermittels eines Einspannrandes 10 in einem Adapterteil 11 aufgenommen ist. Ein freier Siebbereich S befindet sich innerhalb des Einspannrandes 10. Das Adapterteil 11 stellt auch einen Teilabschnitt des Saugluftkanals 6. Wie sich insbesondere auch aus den Figuren 2 und 3 ergibt, weist das Siebteil 9 einen Vorstandsbereich 12 auf, der eine Abflachung 13 besitzt.

Der Vorstandsbereich 12 ist um ein Vorstandsmaß v aus der Ebene E, die durch den Einspannrand 10 in den Bereich geht, in dem der Siebbereich S in diesen übergeht, herausgehoben. Das Vorstandsmaß v entspricht 10 bis 20% der Abmessung einer Querschnittsabmessung L (als gerade Querschnittslinie gesehen). Weiter bevorzugt etwa 15%. In die Offenbarung des genannten Bereichs von 10 bis 20% sind auch alle Zwischenwerte, insbesondere in 1/10% - Schritten, im Hinblick auch auf eine Einengung der Bereichsangabe von unten und/oder oben um jeweils 1/10 oder mehr Prozent, eingeschlossen.

Das Siebteil 9 ist insgesamt aus einem Drahtgewebe gebildet, wobei die Querschnittsdarstellungen der Fig. 1 bis 3 und 4, 6 jeweils einen Drahtmittig durchschneiden.

Das Siebteil 9 ist weiterhin kreisförmig gestaltet.

Wie sich ebenfalls insbesondere aus den Fig. 2 und 3 ergibt, kann die Abwinklung des Einspannrandes 10 einmal entgegengesetzt zu dem Vorspannbereich 12 und zum anderen auch in Richtung der durch den Vorstandsbereich 12 gegebenen Auswölbung des Siebteils 9 geformt sein. Der Einspannrand 10 kann eine Umspritzung des Drahtgewebes aufweisen, kann aber auch nur durch das Drahtgewebe selbst gebildet sein.

Für die betriebliche Wirksamkeit des Inhalators ist wesentlich, dass der Abstand a, siehe etwa Fig. 3, zwischen einem oberen Ende des unbewegten, in einer Ausgangsstellung befindlichen Substanz-Vorratsbehältnisses 8 und dem insofern nächstliegenden Bereich, hier der Abflachung 13, des Siebteils 9, sehr genau definiert ist und auch in Anbetracht von Herstellungstoleranzen, insbesondere des Siebteils 9 sehr genau eingehalten werden kann. Dies ist durch die Abflachung 13 gegeben. Angestrebt ist eine Maßtoleranz die der Hälfte oder weniger der Drahtdicke des Siebteils 9 entspricht.

Zudem kommt es bei Berührungen zwischen dem Substanz-Vorratsbehältnis 8 und dem Siebteil 9 auf Grund der damit gegebenen stabileren Gestaltung auch weniger zu unerwünschten Beeinträchtigungen der Maßhaltigkeit.

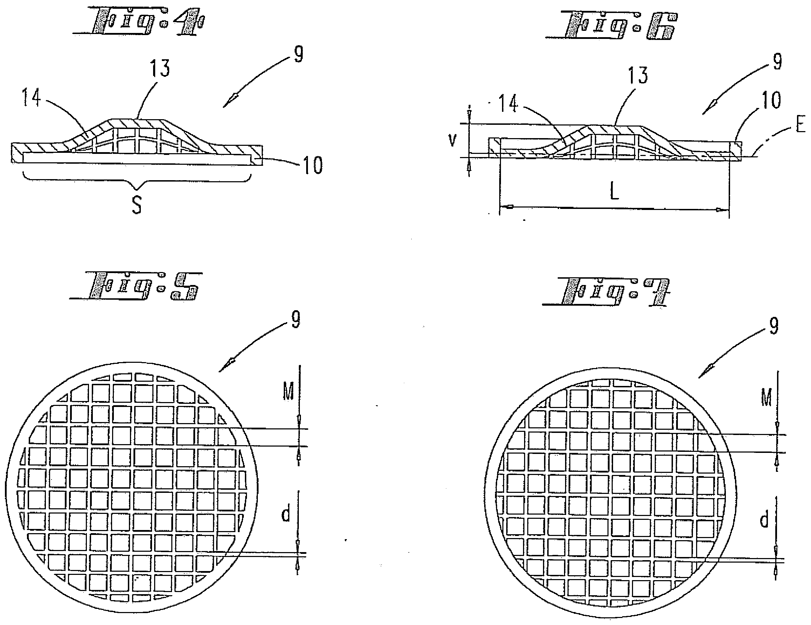

Wie in weiterer Einzelheit auch Fig. 4 und Fig. 6 zu entnehmen ist, schließt sich an die Abflachung 13 nach radial außen ein annähernd gerade verlaufender Übergangsbereich 14, der entsprechend der grundsätzlichen Kreisform des Siebteils 9 umlaufend gegeben ist und damit kegelförmig ist, an.

Die Maschenweite M, wie sie in Bezug auf Fig. 5 oder Fig. 7 dargestellt ist, liegt bei 0,8 mm, während eine Dicke d eines Drahtes, wie er für das Siebgewebe verwendet ist, bei 0,25 mm liegt.

Alle offenbarten Merkmale sind (für sich) erfindungswesentlich.

## Patentansprüche

1. Siebteil (9) für einen Inhalator (1), wobei das Siebteil (9) insgesamt aus einem Drahtgewebe gebildet ist und einen Einspannrand (10) und einen in einem Querschnitt innerhalb des Einspannrandes (10) verlaufenden Siebbereich aufweist, wobei der Einspannrand (10) nur durch das Drahtgewebe selbst gebildet ist und der Siebbereich einen zu einer Seite hin ausladend geformten Vorstandsbereich (12) aufweist,
**dadurch gekennzeichnet, dass**
der Einspannrand (10) eine Abwinklung aufweist, wobei die Abwinklung durch einen äußersten Randabschnitts des Siebteils (9) gebildet wird, wobei die Abwinklung entweder entgegengesetzt zur Richtung des Vorstandsbereichs (12) oder in Richtung des Vorstandsbereichs (12) ausgebildet ist.

2. Siebteil (9) nach Anspruch 1, **dadurch gekennzeichnet, dass** der Siebbereich oder das Siebteil (9) insgesamt aus metallischen Drähten gefertigt ist.

3. Siebteil (9) nach Anspruch 2, **dadurch gekennzeichnet, dass** der Siebbereich eine Maschenweite von mindestens 0,4 mm aufweist.

4. Siebteil (9) nach Anspruch 2 oder 3, **dadurch gekennzeichnet, dass** der Siebbereich eine Maschenweite von 1,5 mm oder weniger aufweist.

5. Siebteil (9) nach Anspruch 2, **dadurch gekennzeichnet, dass** der Draht einen Durchmesser zwischen 0,1 und 0,5 mm aufweist.

6. Siebteil (9) nach Anspruch 1, **dadurch gekennzeichnet, dass** der Vorstandsbereich (12) eine Abflachung (13) aufweist, wobei die Abflachung (13) in der Querschnittsbetrachtung vorzugsweise mittig bezüglich des Siebbereiches ist.

7. Siebteils (9) nach einem der vorhergehenden Ansprüche, **dadurch gekennzeichnet, dass** das Siebteil (9) aus einem Drahtgewebe-Flachstück durch Umbiegen oder Tiefziehen, unter zumindest teilweise plastischer Verformung, hergestellt ist.

8. Inhalator (1) für pulverförmige Substanzen, mit einem zu einem Mundstück (4) führenden Saugluftkanal (6), weiter einem in einer Aufnahmekammer (7) vorgesehenen Substanz-Vorratsbehältnis (8) und einem Siebteil (9) nach einem der Ansprüche 1 bis 6, wobei das Siebteil (9) in dem Saugluftkanal (6) zwischen der Aufnahmekammer (7) und dem Mundstück (4) angeordnet ist.

## Claims

1. Sieve part (9) for an inhaler (1), the sieve part (9) being formed entirely from a wire mesh and having a retaining edge (10) and a sieve area extending over a cross-sectional area within the retaining edge (10), the retaining edge (10) being formed only by the wire mesh itself, and the sieve area having a protruding area (12) which is shaped to project to one side, **characterised in that** the retaining edge (10) has an angular bend, the angular bend being formed by an outermost edge portion of the sieve part (9), the angular bend either being formed opposite to the direction of the protruding area (12) or in the direction of the protruding area (12).

2. Sieve part (9) according to claim 1, **characterised in that** the sieve area or the sieve part (9) as a whole is made of metal wires.

3. Sieve part (9) according to claim 2, **characterised in that** the sieve area has a mesh size of at least 0.4 mm.

4. Sieve part (9) according to either claim 2 or claim 3, **characterised in that** the sieve area has a mesh size of 1.5 mm or less.

5. Sieve part (9) according to claim 2, **characterised in that** the wire has a diameter of between 0.1 and 0.5 mm.

6. Sieve part (9) according to claim 1, **characterised in that** the protruding area (12) has a flat portion (13), the flat portion (13) being preferably central with respect to the sieve area when viewed in cross section.

7. Sieve part (9) according to any of the preceding claims, **characterised in that** the sieve part (9) is produced from a flat piece of wire mesh by means of bending or deep drawing, under at least partial plastic deformation.

8. Inhaler (1) for powdered substances, having a suction air channel (6) leading to a mouthpiece (4), furthermore a substance supply container (8) arranged in a receiving chamber (7), and a sieve part (9) according to any of claims 1 to 6, the sieve part (9) being disposed in the suction air channel (6) between the receiving chamber (7) and the mouthpiece (4).

## Revendications

1. Élément filtrant (9) pour un inhalateur (1), l'élément filtrant (9) se composant globalement d'un tissu de fil métallique et présentant un bord de serrage (10) et une zone de filtrage s'étendant dans une section transversale dans le bord de serrage (10), le bord de serrage (10) étant composé seulement par le tissu de fil métallique lui-même et la zone de filtrage présentant une zone débordante (12) formée en saillie vers un côté,
**caractérisé en ce que**
le bord de serrage (10) présente un coude, le coude étant composé par une section de bord la plus extérieure de l'élément filtrant (9), le coude étant réalisé à l'opposé du sens de la zone débordante (12) ou en direction de la zone débordante (12).

2. Élément filtrant (9) selon la revendication 1, **caractérisé en ce que** la zone de filtrage ou l'élément filtrant (9) est globalement fabriqué en fils métalliques.

3. Élément filtrant (9) selon la revendication 2, **caractérisé en ce que** la zone de filtrage présente une ouverture de mailles d'au moins 0,4 mm.

4. Élément filtrant (9) selon la revendication 2 ou 3, **caractérisé en ce que** la zone de filtrage présente une ouverture de mailles d'1,5 mm ou moins.

5. Élément filtrant (9) selon la revendication 2, **caractérisé en ce que** le fil métallique présente un diamètre compris entre 0,1 et 0,5 mm.

6. Élément filtrant (9) selon la revendication 1, **caractérisé en ce que** la zone débordante (12) présente un aplatissement (13), l'aplatissement (13) étant de préférence au milieu par rapport à la zone de filtrage, vu en section transversale.

7. Élément filtrant (9) selon l'une quelconque des revendications précédentes, **caractérisé en ce que** l'élément filtrant (9) est fabriqué en une pièce plate de tissu de fil métallique par repliage ou emboutissage, sous déformation au moins partiellement plastique.

8. Inhalateur (1) pour substances poudreuses, avec un canal d'aspiration d'air (6) menant à un embout buccal (4), en outre avec un réservoir de stockage de substance (8) prévu dans une chambre de réception (7) et un élément filtrant (9) selon l'une quelconque des revendications 1 à 6, l'élément filtrant (9) étant disposé dans le canal d'aspiration d'air (6) entre la chambre de réception (7) et l'embout buccal (4).
